# EUROPEAN PATENT APPLICATION

(11) **EP 2 412 363 A1**
(43) Date of publication of application: **01.02.2012**
(21) Application number: 10755849.6
(22) Date of filing: 09.03.2010
(51) Int. Cl.: A61K 8/39, A61K 8/04, A61K 8/891, A61Q 19/00, C08K 5/5419, C08L 71/02

(54) **SOLUBILIZED COMPOSITION**

(30) Priority: 26.03.2009 JP 2009076158
(71) Applicant: Shiseido Company, Ltd., Chuo-ku Tokyo 104-8010 (JP)
(72) Inventor: TESHIGAWARA, Takashi, Yokohama-shi Kanagawa 224-8558 (JP); ARAKI, Hidefumi, Yokohama-shi Kanagawa 224-8558 (JP); WATANABE, Kei, Yokohama-shi Kanagawa 224-8558 (JP)
(74) Representative: Lippert, Stachow & Partner
(86) International application number: PCT/JP2010/053857
(87) International publication number: WO 2010/110048

(57) **Abstract**

This invention provides a solubilized composition in which a cyclic or straight silicone oil is solubilized in a large amount and stably. The solubilized composition is **characterized by** comprising (a) a polyoxyethylene alkyl ether type nonionic surfactant represented by the following formula (I):

R¹-O-(CH₂CH₂O)ₐ-H (I)

wherein R¹ represents a branched chain alkyl group having 8 to 18 carbon atoms; a represents an integer satisfying 3 ≤a ≤ 20, (b) one or more silicone(s) selected from a cyclic silicone represented by the following formula (II) and a short chain straight silicone represented by the following formula (III):

[(CH₃)HSi-O-]ₘ (II)

(CH₃)₃Si-O-[Si(CH₃)₂O]ₙSi(CH₃)₃ (III)

wherein m represents an integer satisfying 3≤ m ≤ 7; and n represents an integer satisfying 0 ≤ n ≤ 7, and (c) water.

## Description

### RELATED APPLICATIONS

This application claims the priority of Japanese Patent Application No. 2009-076158 filed on March 26, 2009, which are incorporated herein by reference.

### FIELD OF THE INVENTION

The present invention relates to a solubilized composition, and in particular to improvement in solubilization of a cyclic or straight silicone in a water system in a large amount and in a stable manner.

### BACKGROUND OF THE INVENTION

A solubilization technique by which a water (solvent-insoluble substance is transparently and uniformly dissolved has been widely used in the fields of cosmetic products, pharmaceuticals, foods, and the like. In general, a surfactant is used for solubilizing a sparingly soluble substance, and a concentration thereof higher than cmc allows a sparingly soluble substance to be incorporated in a micelle and to be dissolved, thereby being solubilized. Making a system be in such a solubilized state allows for provision of thermodynamic stability to a composition.

In particular, for cosmetic products, upon producing a water-based cosmetic such as a transparent lotion or a serum, an EO or PO addition type nonionic surfactant is added in an appropriate amount as a solubilizing agent for the purpose of solubilizing an oil agent such as a fragrance composed of ester, aldehyde, ketone, alcohol, ether, phenol, lactone, or the like in an aqueous solvent.

Meanwhile, in recent years, silicone oils, which have a high thermal stability and safety as well as various characteristics, and also have an excellent feeling after use, have frequently been used as oil agents. Among the silicone oils, a cyclic or straight silicone oil having a short chain siloxane structure is particularly expected to be added to a water-based cosmetic in that the oil has a high volatility and provides a smooth feeling after use for the skin.

As one for transparently solubilizing a silicone oil, also studied are a transparent silicone composition using a nonionic surfactant such as a specific fatty alcohol (Patent Document 1:Japanese unexamined patent publication No. 62-34955) and a composition using a specific polyether-modified silicone (Patent Document 2: Japanese unexamined patent publication No. 6-56623).

### DISCLOSURE OF THE INVENTION

### PROBLEM TO BE SOLVED BY THE INVENTION

In general, since the amount of an oil agent solubilized depends greatly on the structure and the amount of a solubilizing agent according to a substance to be solubilized as well as the HLB thereof, there has been desired a solubilizing agent capable of transparently solubilizing, in particular, a cyclic or straight silicone oil having a short chain siloxane structure in water more stably and in a large amount.

The present invention has been made in view of such problems of the prior art, and an object thereof is to provide a solubilized composition in which a cyclic or straight silicone oil is solubilized in a large amount and stably.

### MEANS TO SOLVE THE PROBLEM

In order to achieve the object, the present inventors and others have made intense studies, and as a result, have found that addition of a nonionic surfactant having a specific structure allows a cyclic or straight silicone oil to be solubilized in water in an extremely large amount to give a transparent to almost transparent aqueous solution excellent in stability, thereby leading to the completion of the present invention.

That is, a solubilized composition according to the present invention is characterized by comprising:
(a) a polyoxyethylene alkyl ether type nonionic surfactant represented by the following formula (I);

   R¹-O-(CH₂CH₂O)ₐ-H (I)

   wherein, R¹ represents a branched chain alkyl group having 8 to 18 carbon atoms, and a represents an integer that satisfies 3 ≤ a ≤ 20,
(b) one or more silicones selected from a cyclic silicone represented by the following formula (II) and a short chain straight silicone represented by the following formula (III);

   [(CH₃)HSi-O-]ₘ (II)

   (CH₃)₃Si-O-[Si(CH₃)₂O]ₙSi(CH₃)₃ (III)

   wherein, m represents an integer satisfying 3 ≤ m ≤ 7; and n represents an integer satisfying 0 ≤ n ≤ 7 and
(c) water.

In addition, in the solubilized composition, it is preferable that, a represent an integer satisfying 8 ≤ a ≤ 15 in (a) the nonionic surfactant.

In addition, in the solubilized composition, it is preferable that, (a) the nonionic surfactant is POE isotridecyl ether.

In addition, in the solubilized composition, it is preferable that, (b) the silicone comprises cyclopentasiloxane and/or dimethicone.

In addition, in the solubilized composition, it is preferable that, the ratio, by weight, of the amount of (b) the silicone to that of (a) the nonionic surfactant added is 1 :0.1 to 1:5.

In addition, a cosmetic according to the present invention is characterized by being made of the solubilized composition.

### EFFECT OF THE INVENTION

According to the present invention, a solubilized composition can be obtained in which a sparingly soluble cyclic/straight silicone is solubilized in water in a large amount and stably. Further, according to the solubilized composition, silicones having the specific structure, which has been conventionally resistant to addition to a water-based cosmetic, can be added stably.

### DESCRIPTION OF THE PREFERRED EMBODIMENT

The nonionic surfactant with a branched chain alkyl group used in the present invention is a polyoxyethylene alkyl ether type nonionic surfactant represented by the following formula (I).

R¹-O-(CH₂CH₂O)ₐ-H (I)

In the formulae, R¹ represents a branched chain alkyl group having 8 to 18 carbon atoms. Specific examples of the branched chain alkyl group having 8 to 18 carbon atoms include an isotridecyl, isocetyl, and isostearyl groups.

In addition, a denotes an average number of moles added of ethyleneoxide (CH₂CH₂O), and is an integer of 3 to 20, more preferably an integer of 8 to 15.

It is to be noted that in the formulae (I), the hydrophilic-lipophilic balance (HLB value) varies according to the average number of moles added of ethyleneoxide denoted by a and the chain lengths of alkyl groups represented by R¹.

In the solubilization of the carboxy-modified silicone in the present invention, a nonionic surfactant is preferably used in which the average number of moles added of ethyleneoxide and the chain lengths of alkyl groups are within the numerical value ranges and the HLB value is 8 or more. If the HLB value is less than 8, the lipophilicity is so high that the surfactant is resistant to dissolution in water in some cases. In such cases, it is preferable to use a surfactant having a HLB value of more than 8 together.

Examples of the nonionic surfactant which has an HLB value of 8 or more and which is particularly preferable in the present invention include POE (3) isotridecyl ether [HLB8], POE (5) isotridecyl ether [HLB10], and POE (8) isotridecyl ether [HLB13].

The nonionic surfactant is easily dissolved in water to be a transparent aqueous solution. Further, a cyclic or straight silicone is added to this solution thereby to be solubilized, which makes it possible to obtain the solubilized composition of the present invention as a transparent to almost transparent aqueous solution.

The silicone which can be solubilized in the nonionic surfactant solution is one or more selected from a cyclic silicone represented by the following formula (II) and a short chain straight silicone represented by the following formula (III).

[(CH₃)HSi-O-)ₘ (II)

(CH₃)₃Si-O-[Si(CH₃)₂O]Si(CH₃)₃ (III)

In the formulae, m and n are each an integer satisfying 3 ≤ m ≤7 and 0 ≤ n ≤ 7, and more preferably an integer satisfying 3 ≤ n ≤ 5 and 0 ≤ m ≤ 2.

That is, the cyclic silicone represented by the formula (II) is methylhydrodiene cyclosiloxane, and examples of cyclosiloxane include cyclotrisiloxane (D3), cyclotetrasiloxane (D4), cyclopentasiloxane (D5), cyclohexasiloxane (D6), and cycloheptasiloxane (D7).

In addition, examples of the short chain straight silicone represented by the formula (III) include hexamethyldisiloxane (dimethicone), octamethyltrisiloxane (trisiloxane), decamethyltetrasiloxane, tetradecamethylhexasiloxane, hexadecamethylheptasiloxane, octadecamethyloctasiloxane, and eicosamethylnonasiloxane. In the solubilized composition according to the present invention, a particularly preferable short chain straight silicone is hexamethyldisiloxane.

The solubilized composition according to the present invention can be prepared by appropriately mixing and dissolving the essential components, that is, a nonionic surfactant with a specific structure having a branched alkyl chain, a cyclic and/or short chain straight silicone, and water under heating. Further, upon manufacturing the present invention, other components such as solvents, for example, ethanol and phenoxyethanol may be added therein.

The amount of each of the components added is preferably as follows: the amount of water is 60% or more by weight relative to the solubilized composition, and the ratio, by weight, of the amount of the cyclic and/or short chain straight silicone to that of the nonionic surfactant added is 1:0.1 to 1:5.

If the amount of water added is too small, the nonionic surfactant is not sufficiently dissolved in the composition in some cases, whereas if the ratio of the silicone to the nonionic surfactant added does not fall within the range, the separation of the system is caused, thereby failing to achieve sufficient solubilization in some cases.

Any component usually available for cosmetic products, quasi drugs, phannaceuticals, or the like can be added to the solubilized composition of the present invention as long as the effect of the present invention is not impaired, to prepare a cosmetic. Examples of such any component are given below. (1) Humectants: polyethyleneglycol, propylene glycol, dipropylene glycol, hexylene glycol, glycerin, diglycerin, xylitol, maltitol, maltose, D-mannitol and the like.

(2) Water-soluble polymers: plant-derived polymer such as gum arabic, carrageenan, pectin, agar, quince seed, starch and algae colloid (brown alga extract); microbially-derived polymer such as dextran and pullulan; animal-derived polymer such as collagen, casein and gelatin; starch-based polymer such as carboxymethyl starch and methylhydroxypropyl starch; alginic acid-based polymer such as alginate sodium; vinyl polymer such as carboxy vinyl polymer (e.g. CARBOPOL); polyoxyethylene polymer; polyoxyethylene/polyoxypropylene copolymer; acrylic polymer such as sodium polyacrylate and polyacrylamide; water-soluble inorganic polymer such as bentonite, magnesium aluminum silicate and laponite; and the like.

(3) Ultraviolet absorbers: benzoic acid type ultraviolet absorber such as para-aminobenzoic acid; anthranilic acid type ultraviolet absorber such as methyl anthranilate; salicylic acid type ultraviolet absorber such as octyl salicylate and phenyl salicylate; cinnamic acid type ultraviolet absorber such as isopropyl para-methoxycinnamate, octyl para-methoxycinnamate and mono 2-ethylhexanoate glyceryl dipara-methoxycinnamate; benzophenone type ultraviolet absorber such as 2,4-dihydroxybenzophenone, 2-hydroxy-4-methoxybenzophenone and 2-hydroxy-4-methoxybenzophenone-5-sulfonic acid; urocanic acid; 2-(2'-hydroxy-5'-methylphenyl) benzotriazole; 4-tert-butyl-4'-methoxybenzoylmethane; and the like.

(4) Sequestrants: sodium salt of edetic acid, sodium metaphosphate, phosphoric acid and the like.

(5) Antioxidants: ascorbic acid, alpha-tocopherol, dibutylhydroxytoluene, butylhydroxyanisol and the like.

(6) Drugs: vitamin such as vitamin A oil, retinal, retinol palmitate, inositol, pyridoxine hydrochloride, benzyl nicotinate, nicotinic-acid amide, dl-alpha-tocopherol nicotinate, ascorbic acid magnesium phosphate, ascorbic acid 2-glucoside, vitamin D2 (ergocalciferol), L-ascorbic acid dl-alpha-tocopherol phosphoric acid diester potassium salt, dl-alpha-tocopherol, dl-alpha-tocopherol acetate, pantothenic acid and biotin; hormone such as estradiol and ethinyl estradiol; anti-inflammatory agent such as allantoin and azulene; skin-lightening agent such as arbutin; astringent agent such as zinc oxide and tannin; algefacient such as L-menthol and camphor; sulfur; lysozyme chloride; pyridoxine hydrochloride; gamma-oryzanol; and the like.

(7) Various extracts: houttuynia extract, phellodendron amurense extract, melilot extract, lamium album extract, licorice (glycyrrhiza glabra) extract, peony root extract, saponaria officinalis extract, luffa cylindrica extract, cinchona succirubra bark extract, saxifraga sarmentosa extract, sophora angustifolia extract, nuphar japonicum extract, fennel extract, primula extract, rose extract, rehmannia chinensis root extract, lemon extract, lithospermum officinale extract, aloe extract, acous calamus root extract, eucalyptus globules extract, horsetail extract, sage extract, thyme extract, tea extract, alga extract, cucumber extract, clove extract, raspberry extract, melissa extract, panax ginseng root extract, carrot extract, horse chestnut extract, peach extract, peach leaf extract, mulberry extract, cornflower extract, hamamelis virginiana extract, silk extract and the like.

(8) Others: powders, pigments, pH adjuster, preservative (e.g. phenoxyethanol) and the like.

The various components can be used not only in a free state but also in an acid or base form, when possible, and an ester derivative thereof can also be used when having a carboxyl group. Further, if necessary, a surfactant other than the essential components can also be added to the solubilized composition of the present invention. Examples of such a surfactant include an anionic surfactant, a cationic surfactant, a nonionic surfactant, and an amphoteric surfactant that are generally added to a cosmetic or the like.

Since the solubilized composition of the present invention is excellent in stability, solubilizing ability, and feeling after use, it is particularly useful as a cosmetic product or a pharmaceutical, and it can be formed into a liquid formulation such as a lotion, an aftershave lotion, a serum, a body lotion, a hair tonic, a hair liquid, a hair restorer, and a fragrance. Further, the solubilized composition of the present invention can also be applied to other formulations as long as the effect of the present invention is not impaired. For example, the solubilized composition of the present invention can also be formed into a gel formulation by using a known thickening agent or the like, a sheet formulation by being impregnated in a nonwoven cloth or the like, or a spray, an aerosol, or a roll-on type formulation by a known method.

### EXAMPLES

Hereinafter, the present invention will be described with reference to specific examples, but it is not to be limited thereto. The amount added is expressed in % by weight, unless otherwise specified. It is to be noted that a test method used below is as follows.

### (Test Example 1)

10% by weight of POE (5) isotridecylether, 0 to 25% by weight of dimethicone, and balance being water were mixed at 70°C under heating, and cooled to 30°C to obtain liquid compositions.

When the resulting compositions each having a different silicone concentration were observed macroscopically immediately after preparation in terms of the appearance thereof, the formation of an extremely transparent single layer system was confirmed in a sample with the amount of dimethicone added being 19 to 20% by weight.

### (Test Examples 2)

10% by mass of a mixture of POE (5) isotridecylether and POE (3) isotridecyl ether (mass ratio: 3:1), 18 to 30% by mass of dimethicone, and balance being water were mixed at 70°C under heating, and cooled to 30°C to obtain liquid compositions.

When the resulting compositions each having a different silicone concentration were observed macroscopically immediately after preparation in terms of the appearance thereof, the formation of an almost transparent single layer system was confirmed in a sample with the amount of dimethicone added being 26 to 27% by weight.

### (Test Examples 3)

10% by weight of POE (8) isotridecyl ether, 0 to 20% by weight of cyclopentasiloxane, and balance being water were mixed at 70°C under heating, and cooled to 30°C to obtain liquid compositions.

When the resulting compositions each having a different silicone concentration were observed macroscopically immediately after preparation in terms of the appearance thereof, the formation of a transparent single layer system was confirmed in a sample with the amount of cyclopentasiloxane added being 1 to 3% by weight.

It has been revealed from the foregoing results in Test Examples 1 to 3 that the use of the polyoxyethylene alkyl ether type nonionic surfactant having a specific structure as a solubilizing agent allows obtaining a composition with a cyclic and/or short chain straight silicone solubilized in water. Further, it has been found from the results of an additional study based on the test that, to form the transparent to almost transparent solubilized composition, the ratio, by weight, of the amount of the cyclic and/or short chain straight silicone to that of the nonionic surfactant added is suitably 1:0.1 to 1:5 (surfactant: silicone).

Hereinafter, formulation examples of the present invention will be described with reference to specific examples, but the present invention is not to be limited thereto.

### Formulation Example 1

| (Component) | (% by mass) |
|---|---|
| POE (4) isotridecyl ether | 2 |
| Hexamethyldisiloxane | 5 |
| Ethanol | 2 |
| Phenoxyethanol | 0.3 |
| Water | Balance |

### (Production method)

POE (4) isotridecyl ether and hexamethyldisiloxane were mixed with a mixture of ethanol, phenoxyethanol and water under high temperature (70 degrees C), and then the product obtained was cooled.

### Formulation Example 2

| (Component) | (% by mass) |
|---|---|
| POE (10) isocetyl ether | 0.5 |
| Trisiloxane | 2 |
| Glycerin | 2 |
| Phenoxyethanol | 0.5 |
| Water | Balance |

### (Production method)

POE (10) isocetyl ether and trisiloxane were mixed with a mixture of glycerin, phenoxyethanol and water under high temperature (70 degrees C), and then the product obtained was cooled.

## Claims

1. A solubilized composition comprising:
(a) a polyoxyethylene alkyl ether type nonionic surfactant represented by the following formula (I):
R¹-O-(CH₂CH₂O)ₐ-H (I)
wherein R¹ represents a branch chain alkyl group having 8 to 18 carbon atoms; a represents an integer satisfying 3 ≤ a ≤ 20,
(b) one or more silicone(s) selected from a cyclic silicone represented by the following formula (II) and a short chain straight silicone represented by the following formula
[(CH₃)HSi-O-]ₘ (II)
(CH₃)₃Si-O-[Si(CH₃)₂O]ₙSi(CH₃)₃ (III)
wherein m represents an integer satisfying 3 ≤ m ≤ 7; and n represents an integer satisfying 0 ≤ n ≤ 7, and
(c) water.

2. The solubilized composition according to claim 1, wherein a is an integer satisfying 8 ≤a≤ 15 in (a) the nonionic surfactant.

3. The solubilized composition according to claim 1 or 2, wherein (a) the nonionic surfactant is POE isotridecyl ether

4. The solubilized composition according to any of claims 1 to 3, wherein (b) the silicone comprises decamethylcycropentasiloxane and/or dimethylpolysiloxane.

5. The solubilized composition according to any of claims 1 to 4, wherein a ratio by weight of the silicone to the nonionic surfactant is 1:0.1 to 1:5.

6. A cosmetic comprising the solubilized composition of any of claims 1 to 6.
